# EUROPÄISCHE PATENTSCHRIFT

(11) **EP 2 033 617 B1**
(45) Veröffentlichungstag und Bekanntmachung des Hinweises auf die Patenterteilung: **06.10.2010**
(21) Anmeldenummer: 08014491.8
(22) Anmeldetag: 14.08.2008
(51) Int. Cl.: A61K 8/02, A61K 8/06, A61K 8/64, A61K 8/81, A61Q 19/08

(54) **Kosmetische Pflegecreme enthaltend Protein**
Cosmetic care cream comprising a protein
Crème de soin cosmétique comprenant une proteine

(30) Priorität: 31.08.2007 DE 102007041491
(43) Veröffentlichungstag der Anmeldung: 11.03.2009
(73) Patentinhaber: Henkel AG & Co. KGaA, 40589 Düsseldorf (DE)
(72) Erfinder: Struwe, Alexandra, 47877 Willich (DE); Waldmann-Laue, Marianne, 40789 Monheim (DE); Frisoli, Christian, 41564 Kaarst (DE)

(56) Entgegenhaltungen:
- EP-A- 1 430 906
- EP-A- 1 514 537
- WO-A-93/11865
- WO-A-99/52557
- WO-A-02/094209
- WO-A-03/022236
- WO-A-2004/000248

## Beschreibung

Die vorliegende Anmeldung betrifft die Verwendung kosmetischer und pharmazeutischer Zusammensetzungen in Form von Öl-in-Wasser-Emulsionen ausgewählter polarer Ölkomponenten, die als Emulgatoren ein wasserlösliches Protein enthalten als Pflegemaske, mit einem verbesserten Hautgefühl. Solche Emulsionen lassen sich ohne die Verwendung üblicher Öl-in-Wasser-Emulgatoren formulieren. Die erfindungsgemäßen Zusammensetzungen weisen eine besonders hohe Hautpflegewirkung auf und beugen dem Risiko einer allergischen Reaktion vor. Die verbesserten Eigenschaften der erfindungsgemäß verwendeten Zusammensetzungen beruhen vor allem auf einer geschmeidigen und gut verteilbaren Konsistenz, verbesserter Lager- und Temperaturstabilität. Weiterhin zeigen die erfindungsgemäß verwendeten Zusammensetzungen ein sehr gutes Anhaften auf der Haut, insbesondere der Gesichtshaut, ohne dass dabei das unerwünschte Phänomen der Migration zu beobachten ist. Andererseits lässt sich die erfindungsgemäß vewendete Zusammensetzung auch einfach wieder von der Haut entfernen und ist damit hervorragend als Basis für Pflegemasken geeignet.

WO 2004/000248 A2 beschäftigt sich mit kosmetischen Zusammensetzungen, die mindestens ein vernetztes Copolymer aus Vinylpyrrolidon und 2-Methyl-2[(1-oxo-2-propenyl)amino]-1-propansulfonsäure, das teilweise oder vollständig neutralisiert ist, sowie ein teilweise oder vollständig neutralisiertes, gegebenenfalls vernetztes Polymer aus mindestens einem der Monomere Acrylsäure, Methacrylsäure, Itaconsäure, Maleinsäure und 2-Methyl-2[(1-oxo-2-propenyl)amino]-1-propansulfonsäure, die neutrale Comonomere, ausgewählt aus Acrylamid und den gewünschtenfalls hydroxylierten C₁ - C₃₀-Alkyl-Estern von Acrylsäure, Methacrylsäure, Itaconsäure und Maleinsäure, enthalten können.

EP 1430906 A2 offenbart kosmetische Zusammensetzungen, die die in einem zur topischen Anwendung geeigneten Träger mindestens ein nanoskaliges Hydrogel aus mindestens einem Polymer mit einem mittleren Teilchendurchmesser von 10 - 1000 nm, das eine LCST (Lower Critical Solution Temperature, untere kritische Entmischungstemperatur) von 28 - 38 °C aufweisen, sowie weiterhin mindestens ein verdickendes Polymer enthalten, das ausgewählt ist aus teilweise oder vollständig neutralisierten, gegebenenfalls vernetzten Polymeren aus mindestens einem der Monomere Acrylsäure, Methacrylsäure, Itaconsäure, Maleinsäure und 2-Methyl-2[(1-oxo-2-propenyl)-amino]-1-propansulfonsäure, das mindestens ein neutrales Comonomer, ausgewählt aus Vinylpyrrolidon, Acrylamid und den C₁-C₃₀-Estern von Acrylsäure, Methacrylsäure, Itaconsäure und Maleinsäure, enthalten kann. WO 2002/094209 A2 offenbart Proteinemulgator-basierte Zusammensetzungen mit UV-Schutz.

### Aufgabenstellung

Der vorliegenden Anmeldung lag die Aufgabe zugrunde, eine kosmetische oder dermatologische Hautpflegezusammensetzung bereitzustellen, die für eine Pflegemaske geeignete Anwendungseigenschaften (leicht aufzutragen, gute Hafteigenschaften, kein Tropfen, trotzdem wieder einfach zu entfernen, keine Migration) aufweist, bei gleichzeitig hoher Lager- und Temperaturstabilität und hervorragender Hautverträglichkeit, insbesondere für empfindliche Haut und/oder für zu allergischen Reaktionen neigende Haut.

Gelöst werden diese Aufgaben durch die erfindungsgemäß verwendeten Zusammensetzungen. Ein erster Gegenstand der vorliegenden Anmeldung ist ein nicht-therapeutisches, kosmetisches Verfahren zur Behandlung der Haut, wobei eine kosmetische Zusammensetzung in Form einer Öl-in-Wasser-Emulsion, enthaltend ein Emulgatorsystem, umfassend 0,1 - 0,3 Gew.-% eines Proteins und 3 - 20 Gew.-% eines Öls, ausgewählt aus pflanzlichen Ölen sowie synthetischen Triglyceriden, synthetischen Propylenglycoldiestern und Fettsäureestern einwertiger linearer oder verzweigter C₂-C₁₈-Alkanole, die unter Normalbedingungen flüssig sind, sowie Mischungen dieser Komponenten, weiterhin 13-20 Gew.-% Konsistenzgeber(n), die unter Normalbedingungen fest sind, ausgewählt aus linearen, gesättigten C₁₂-C₃₀-Alkanolen, Wachsen und Partialestern von C₂ - C₁₀-Polyolen mit C₈-C₃₀-Fettsäuren, sowie Mischungen dieser Komponenten,
ein Polymerverdickersystem, umfassend mindestens ein teilweise oder vollständig neutralisiertes, vernetztes Copolymer aus 2-Methyl-2[(1-oxo-2-propenyl)amino]-1-propansulfonsäure (AMPS) und Vinylpyrrolidon, sowie weiterhin mindestens ein teilweise oder vollständig neutralisiertes, vernetztes Copolymer aus 2-Methyl-2[(1-oxo-2-propenyl)amino]-1-propansulfonsäure (AMPS) und mindestens einem weiteren Monomer, ausgewählt aus Acrylsäure und/oder einem Acrylsäuresalz, auf die Haut, insbesondere die Gesichtshaut, aufgetragen wird, dort für eine Zeit von 5 bis 60 Minuten verbleibt und anschließend mit Wasser und/oder einem Substrat, insbesondere einem Lappen, Tuch, Pad oder Bausch, entfernt wird.

### Normalbedingungen

Alle Angaben über die Aggregatzustände der verwendeten Ausgangsstoffe (fest, flüssig...) in dieser Anmeldung beziehen sich auf Normalbedingungen. "Normalbedingungen" sind im Sinne der vorliegenden Anmeldung eine Temperatur von 20°C und ein Druck von 1013,25 mbar. Schmelzpunktangaben beziehen sich ebenfalls auf einen Druck von 1013,25 mbar.

### Wasserlöslichkeit

Erfindungsgemäß wird unter Wasserlöslichkeit eine Löslichkeit von wenigstens 5 Gew.-% bei 20°C verstanden. 5 Gew.-% bedeutet, dass 5 g einer Substanz in 95 g Wasser bei 20°C löslich sind.

### Proteine

Das mindestens eine erfindungsgemäß verwendete Protein ist wasserlöslich. Das mindestens eine erfindungsgemäß verwendete Protein bewirkt in wässriger Lösung gegenüber ausgewählten Ölkomponenten eine Erniedrigung der Grenzflächenspannung Öl/Wasser und dient aufgrund dieser Grenzflächenaktivität als Emulgator. Bevorzugt sind solche Proteine enthalten, die in 1 Gew.-%iger Lösung bei 25° C die Grenzflächenspannung des Wassers zur Ölkomponente um einen Betrag (γ¹ γ²) von wenigstens 2 mN/m senken.

Bevorzugte Proteine, die diese Anforderungen im Zusammenwirken mit den erfindungsgemäß ausgewählten Ölkomponenten erfüllen, sind pflanzlicher Herkunft. Besonders bevorzugt stammen die emulgierenden Proteine aus Pflanzensamen. Erfindungsgemäß bevorzugt sind insbesondere Mandel-, Haselnuss- und Baumwollsamenprotein sowie Proteine aus den Samen von Hibiscus esculentus (Okra). Weitere geeignete Proteine und Proteinhydrolysate werden aus Leguminosen, z. B. aus Erbsen, und aus Getreide, insbesondere aus Hafer, Weizen und Soja, gewonnen.

Bevorzugt sind Proteine aus Erbsen, Hafer, Weizen und den Samen von Hibiscus esculentus. Ganz besonders bevorzugte Proteine stammen aus den Samen von Hibiscus esculentus und aus Erbsen.

Der Gehalt an emulgierendem Protein beträgt 0,1 - 0,3 Gew.-%, bevorzugt 0,15 - 0,25 Gew.-%, jeweils bezogen auf die Gesamtzusammensetzung.
Die erfindungsgemäß besonders bevorzugten Proteine enthalten Globuline mit einem Molgewicht von mehr als 30.000 Dalton in Anteilen von 50 - 90 %, bevorzugt 60 - 85 % und Albumine mit einem Molgewicht unter 30.000 Dalton in Anteilen von 10 - 50%, bevorzugt 15 - 40 %, bezogen auf den Proteingehalt.
Ein besonders bevorzugtes Erbsenprotein ist als Produkt "V-Protein liquid" von Cosmetochem erhältlich und enthält 18 % Albumine (Molgewicht 15.000 bis 30.000 Dalton) sowie 82 % Globuline (Molgewicht 150.000 bis 300.000 Dalton). Dieser Rohstoff weist einen Aktivsubstanzgehalt von 1,5 - 3 Gew.-% Protein auf. Ein besonders bevorzugtes Protein aus den Samen von Hibiscus esculentus ist als Produkt Hibiscin^{®} HP LS 9198 von Laboratoires Sérobiologiques erhältlich, das ein durchschnittliches Molgewicht von etwa 500.000 Dalton und folgende Zusammensetzung der Molgewichtsfraktionen aufweist:
- größer als 500.000 Dalton : 48,4 %
- 100.000 bis 500.000 Dalton: 4.8 %
- 30.000 bis 100.000 Dalton: 8.5 %
- 5000 bis 30.000 Dalton: 22,0 %
- kleiner als 5000 Dalton: 16,3 %.

In einer bevorzugten Ausführungsform enthalten die erfindungsgemäßen Zusammensetzungen, bezogen auf die Gesamtzusammensetzung, 30 bis 75 Gew.-% Wasser, besonders bevorzugt 35 - 65 Gew.-% Wasser, außerordentlich bevorzugt 40 - 50 Gew.-% Wasser.

### Öle

Die zur Emulgierung durch Proteine bevorzugten Ölkomponenten sind ausgewählt aus pflanzlichen Ölen sowie synthetischen Triglyceriden und synthetischen Propylenglycoldiestern, die unter Normalbedingungen flüssig sind, sowie Mischungen dieser Komponenten. Sie sind in den erfindungsgemäßen Zusammensetzungen in einer Gesamtmenge von 3 - 20 Gew.-%, bevorzugt 5 - 18 Gew.-%, besonders bevorzugt 10 - 17 Gew.-%, außerordentlich bevorzugt 14 - 16 Gew.-%, enthalten, jeweils bezogen auf das Gewicht der gesamten Zusammensetzung.

Erfindungsgemäß bevorzugte pflanzliche Öle sind Avocadoöl, Sonnenblumenöl, Sojaöl, Sesamöl, Haselnussöl, Mandelöl sowie Distelöl (Safloröl) und Mischungen hiervon. Besonders bevorzugt ist Distelöl allein sowie Mischungen aus Distelöl und Avocadoöl. Weniger geeignet ist z. B. Nachtkerzenöl (evening primrose oil), dessen Polarität so hoch ist, dass es selbst eine beträchtliche Grenzflächenaktivität zeigt. Erfindungsgemäß außerordentlich bevorzugt sind weiterhin synthetische Fettsäuretriglycerid-Öle. Besonders bevorzugt sind die Triglyceride von gesättigten C₈-C₁₀-Fettsäuren, wobei die Fettsäuren sowohl unverzweigt, wie z. B. bei den Handelsprodukten Myritol^{®} 318 und Myritol^{®} 331 (Cognis) oder Miglyol^{®} 812 (Hüls), als auch verzweigt sein können, wie z. B. bei den Handelsprodukten Estol^{®} GTEH 3609 (Uniqema) oder Myritol^{®} GTEH (Cognis). Weitere bevorzugte Ester dieses Typs sind die Fettsäureester des 1,2-Propylenglycols, des Neopentylglycols, z. B. Neopentylglycoldiheptanoat, des Trimethylolpropans und des Pentaerythrits, wobei die 1,2-Propylenglycolester bevorzugt sind. Beispiele für erfindungsgemäß bevorzugte Ölkomponenten sind die 1,2-Propylenglycoldiester von gesättigten, unverzweigten C₈-C₁₀-Fettsäuren, besonders bevorzugt die Handelsprodukte Myritol^{®} PC und Miglyol^{®} 840.
Beispiele für erfindungsgemäß besonders geeignete Fettsäureester einwertiger linearer oder verzweigter C₂-C₁₈-Alkanole sind Octylethylhexanoat, Isopropylpalmitat, Hexyllaurat, Isopropylmyristat, ein Gemisch der Ester von Caprylsäure und Caprinsäure mit C₁₂-C₁₈-Fettalkoholen, das als Handelsprodukt Cetiol^{®} LC (Cognis) erhältlich ist, C₁₂-C₁₅-Alkyloctanoat, Isodecylneopentanoat, Isononylisononanoat, 2-Ethylhexylpalmitat (z. B. Cegesoft^{®} C 24 von Cognis), 2-Ethylhexylstearat, Cetearylisononanoat (Mischung aus Cetylisononanoat und Stearylisononanoat, z. B. Cetiol^{®} SN (Cognis)), Cetearyloctanoat, Ethylstearat, Isopropylstearat, Butylstearat und Myristylpropionat. Weiterhin geeignet sind die Monoester des Isosorbid (1,4:3,6-Dianhydro-D-glucit) mit C₂-C₂₄-Fettsäuren, z. B. das Isosorbidmonolaurat.
Außerordentlich bevorzugte Ölmischungen umfassen Distelöl, Avocadoöl, 1,2-Propylenglycoldicaprylat, 1,2-Propylenglycoldicaprat und mindestens einen Isopropylester, insbesondere Isopropylpalmitat, Isopropylmyristat oder Isopropylstearat.

### Konsistenzgeber

Die erfindungsgemäß verwendeten Zusammensetzungen enthalten weiterhin 13 - 20 Gew.-% mindestens einer Konsistenz gebenden Substanz, die unter Normalbedingungen fest ist und die ausgewählt ist aus linearen, gesättigten C₁₂-C₃₀-Alkanolen, Wachsen und Partialestern von C₂ - C₁₀-Polyolen mit C₈-C₃₀-Fettsäuren, sowie Mischungen dieser Komponenten.
Bevorzugte Partialester aus einem Polyol mit 2 - 10 C-Atomen und linearen gesättigten und ungesättigten Fettsäuren mit 8 - 30, insbesondere 14 - 22 C-Atomen, die hydroxyliert sein können, sind insbesondere die Mono- und Diester von Glycerin oder Ethylenglycol oder die Monoester von Propylenglycol mit linearen gesättigten und ungesättigten C₁₂ - C₃₀-Carbonsäuren, die hydroxyliert sein können, insbesondere diejenigen mit Palmitin- und Stearinsäure, die Sorbitanmono-, -di- oder - triester von linearen gesättigten und ungesättigten C₁₂ - C₃₀-Carbonsäuren, die hydroxyliert sein können, insbesondere diejenigen von Myristinsäure, Palmitinsäure, Stearinsäure oder von Mischungen dieser Fettsäuren, die Pentaerythritylmono-, -di-, -tri- und -tetraester und die Methylglucosemono- und -diester von linearen, gesättigten und ungesättigten C₁₂ - C₃₀-Carbonsäuren, die hydroxyliert sein können, wovon besonders bevorzugt sind die Mono-, Di-, Tri- und Tetraester von Pentaerythrit mit linearen gesättigten Fettsäuren mit 12 - 30, insbesondere 14 - 22 Kohlenstoffatomen, die hydroxyliert sein können, sowie Mischungen hiervon. Erfindungsgemäß besonders bevorzugt sind die Mono- und Diester. Erfindungsgemäß bevorzugte C₁₂-C₃₀-Fettsäurereste sind ausgewählt aus Laurinsäure-, Myristinsäure-, Palmitinsäure-, Stearinsäure-, Arachinsäure- und Behensäure-Resten; besonders bevorzugt ist der Stearinsäurerest. Erfindungsgemäß bevorzugte Konsistenzgeber sind die Glycerinmono- und -diester der Stearinsäure und der Palmitinsäure.
Weitere bevorzugte Konsistenzgeber sind ausgewählt aus Wachsen.
Generell sind Wachse von fester bis brüchig harter Konsistenz, grob bis feinkristallin, durchscheinend bis opak, jedoch nicht glasartig, und schmelzen oberhalb von 50 °C ohne Zersetzung. Sie sind schon wenig oberhalb des Schmelzpunktes niedrigviskos und zeigen eine stark temperaturabhängige Konsistenz und Löslichkeit.
Erfindungsgemäß bevorzugt sind beispielsweise natürliche pflanzliche Wachse, z. B. Candelillawachs, Carnaubawachs, Japanwachs, Zuckerrohrwachs, Ouricourywachs, Korkwachs, Sonnenblumenwachs, Fruchtwachse wie Orangenwachse, Zitronenwachse, Grapefruitwachs, und tierische Wachse, z. B. Bienenwachs, Schellackwachs und Walrat. Im Sinne der Erfindung kann es besonders bevorzugt sein, hydrierte oder gehärtete Wachse einzusetzen. Als Wachskomponente sind auch chemisch modifizierte Wachse, insbesondere die Hartwachse, wie z. B. Montanesterwachse, hydrierte Jojobawachse und Sasolwachse, einsetzbar. Zu den synthetischen Wachsen, die ebenfalls erfindungsgemäß bevorzugt sind, zählen beispielsweise Polyalkylenwachse und Polyethylenglycolwachse, C₂₀-C₄₀-Dialkylester von Dimersäuren, C₃₀₋₅₀-Alkylbienenwachs sowie Alkyl- und Alkylarylester von Dimerfettsäuren.
Eine besonders bevorzugte Wachskomponente ist ausgewählt aus mindestens einem Ester aus einem gesättigten, einwertigen C₁₆-C₆₀-Alkohol und einer gesättigten C₈-C₃₆-Monocarbonsäure. Erfindungsgemäß zählen hierzu auch Lactide, die cyclischen Doppelester von α-Hydroxycarbonsäuren der entsprechenden Kettenlänge. Ester aus Fettsäuren und langkettigen Alkoholen haben sich für die erfindungsgemäße Zusammensetzung als besonders vorteilhaft erwiesen, weil sie ausgezeichnete sensorische Eigenschaften und insgesamt eine hohe Stabilität verleihen. Die Ester setzen sich aus gesättigten, verzweigten oder unverzweigten Monocarbonsäuren und gesättigten, verzweigten oder unverzweigten einwertigen Alkoholen zusammen. Auch Ester aus aromatischen Carbonsäuren bzw. Hydroxycarbonsäuren (z. B. 12-Hydroxystearinsäure) und gesättigten, verzweigten oder unverzweigten Alkoholen sind erfindungsgemäß einsetzbar. Besonders bevorzugt ist, die Wachskomponenten zu wählen aus der Gruppe der Ester aus gesättigten, verzweigten oder unverzweigten Alkancarbonsäuren einer Kettenlänge von 12 bis 24 C-Atomen und den gesättigten, verzweigten oder unverzweigten Alkoholen einer Kettenlänge von 16 bis 50 C-Atomen, die einen Schmelzpunkt > 50°C haben. Insbesondere können als Wachskomponente C₁₆₋₃₆-Alkylstearate und C1₈₋₃₈-Alkylhydroxystearoylstearate, C₂₀₋₄₀-Alkylerucate sowie Cetearylbehenat vorteilhaft sein. Das Wachs oder die Wachskomponenten weisen einen Schmelzpunkt > 50°C, bevorzugt > 60°C, auf.

Eine besonders bevorzugte Ausführungsform der Erfindung enthält als Wachskomponente ein C₂₀-C₄₀-Alkylstearat. Dieser Ester ist unter den Namen Kesterwachs^{®} K82H oder Kesterwachs^{®} K80H bekannt und wird von Koster Keunen Inc. vertrieben. Es handelt sich um die synthetische Nachahmung der Monoesterfraktion des Bienenwachses und zeichnet sich durch seine Härte, seine Ölgelierfähigkeit und seine breite Kompatibiltät mit Lipidkomponenten aus.

Weitere bevorzugte Wachskomponenten sind die Triglyceride gesättigter und gegebenenfalls hydroxylierter C₁₂₋₃₀-Fettsäuren, wie gehärtete Triglyceridfette (hydriertes Palmöl, hydriertes Kokosöl, hydriertes Rizinusöl), Glyceryltribehenat (Tribehenin) oder Glyceryltri-12-hydroxystearat, weiterhin synthetische Vollester aus Fettsäuren und Glycolen oder Polyolen mit 2 - 6 Kohlenstoffatomen, solange sie einen Schmelzpunkt oberhalb von 50°C aufweisen, beispielsweise bevorzugt C₁₈ - C₃₈ Acid Triglyceride (Syncrowax^{®} HGL-C). Weitere bevorzugte Wachskomponenten sind die gesättigten linearen C₁₄ - C₃₆-Carbonsäuren, insbesondere Myristinsäure, Palmitinsäure, Stearinsäure und Behensäure sowie Mischungen dieser Verbindungen, z. B. Syncrowax**^{®}** AW 1C (C₁₈ - C₃₆-Fettsäuren) oder Cutina^{®} FS 45 (Palmitin- und Stearinsäure).

Weitere äußerst bevorzugte Konsistenzgeber sind ausgewählt aus den gesättigten linearen Fettalkoholen, insbesondere aus Laurylalkohol, Isotridecylalkohol, Myristylalkohol, Cetylalkohol, Stearylalkohol, Arachylalkohol, Behenylalkohol, Erucylalkohol und Brassidylalkohol sowie deren technischen Mischungen. Besonders bevorzugt sind Mischungen aus Cetylalkohol, Stearylalkohol und Behenylalkohol.

Weitere bevorzugte Konsistenzgeber sind ausgewählt aus Wachskomponenten mit einem Schmelzpunkt im Bereich von 25 - < 50°C, ausgewählt aus Kokosfettsäureglycerinmono-, -di- und -triestern, Butyrospermum Parkii (Shea Butter) und Estern von gesättigten, einwertigen C₈-C₁₈-Alkoholen mit gesättigten C₁₂-C₁₈-Monocarbonsäuren sowie Mischungen dieser Substanzen, enthalten ist. Diese niedriger schmelzenden Wachskomponenten ermöglichen eine Konsistenzoptimierung des Produktes. Besonders bevorzugt sind Handelsprodukte mit der INCI-Bezeichnung Cocoglycerides, insbesondere die Handelsprodukte Novata^{®} (ex Cognis), besonders bevorzugt Novata^{®} AB, ein Gemisch aus C₁₂-C₁₈-Mono-, Di- und Triglyceriden, das im Bereich von 30 - 32°C schmilzt, sowie die Produkte der Softisan-Reihe (Sasol Germany GmbH) mit der INCI-Bezeichnung Hydrogenated Cocoglycerides, insbesondere Softisan 100, 133, 134, 138, 142. Weitere bevorzugte Ester von gesättigten, einwertigen C₁₂-C₁₈-Alkoholen mit gesättigten C₁₂-C₁₈-Monocarbonsäuren sind Stearyllaurat, Cetearylstearat (z. B. Crodamol^{®} CSS), Cetylpalmitat (z. B. Cutina^{®} CP) und Myristylmyristat (z. B. Cetiol^{®} MM).

Bevorzugte erfindungsgemäß verwendete Zusammensetzungen sind dadurch gekennzeichnet, dass der mindestens eine Konsistenzgeber, der unter Normalbedingungen fest ist, ausgewählt aus linearen, gesättigten C₁₂-C₃₀-Alkanolen, Wachsen und Partialestern von C₂ - C₁₀-Polyolen mit C₈-C₃₀-Fettsäuren, sowie Mischungen dieser Komponenten, in einer Gesamtmenge von 13 - 20 Gew.-%, bevorzugt 15 - 18 Gew.-%, besonders bevorzugt 17-18 Gew.-%, jeweils, bezogen auf die gesamte Zusammensetzung, enthalten ist.

Die erfindungsgemäß verwendeten Zusammensetzungen sind weiterhin dadurch gekennzeichnet, dass mindestens ein teilweise oder vollständig neutralisiertes, vernetztes Copolymer aus 2-Methyl-2[(1-oxo-2-propenyl)amino]-1-propansulfonsäure (AMPS) und Vinylpyrrolidon enthalten ist. Erfindungsgemäß außerordentlich bevorzugt sind teilweise oder vollständig neutralisierte, vernetzte Copolymere aus 2-Methyl-2[(1-oxo-2-propenyl)amino]-1-propansulfonsäure (AMPS) und Vinylpyrrolidon, die in Form des Ammoniumsalzes kommerziell erhältlich sind, z. B. unter dem Handelsnamen Aristotlex^{®} AVC von der Firma Clariant. Weitere bevorzugte Copolymere aus 2-Methyl-2[(1-oxo-2-propenyl)amino]-1-propansulfonsäure (AMPS) und Vinylpyrrolidon liegen in Form einer Mischung aus Alkalimetallsalzen und Ammoniumsalzen vor, insbesondere bevorzugt als Mischungen der Natrium- und der Ammoniumsalze. Bevorzugt ist das mindestens eine teilweise oder vollständig neutralisierte, vernetzte Copolymer aus 2-Methyl-2[(1-oxo-2-propenyl)amino]-1-propansulfonsäure (AMPS) und Vinylpyrrolidon in einer Gesamtmenge von mindestens 0,1 Gew.-%, besonders bevorzugt von mindestens 0,2 Gew.-%, außerordentlich bevorzugt von mindestens 0,3 Gew.-%, jeweils bezogen auf die gesamte Zusammensetzung, enthalten. Die bevorzugte Obergrenze für dieses Copolymer liegt bei 1 Gew.-%, besonders bevorzugt bei 0,5 Gew.-%.

Die erfindungsgemäß verwendeten Zusammensetzungen sind weiterhin dadurch gekennzeichnet, dass mindestens ein teilweise oder vollständig neutralisiertes, vernetztes Copolymer aus 2-Methyl-2[(1-oxo-2-propenyl)amino]-1-propansulfonsäure (AMPS) und mindestens einem weiteren Monomer, ausgewählt aus Acrylsäure und/oder einem Acrylsäuresalz, enthalten ist. Außerordentlich bevorzugt ist ein teilweise oder vollständig neutralisiertes, vernetztes Copolymer aus 2-Methyl-2[(1-oxo-2-propenyl)amino]-1-propansulfonsäure (AMPS) und Natriumacrylat. Copolymere aus AMPS und Natriumacrylat sind in Form eines inversen Latex kommerziell erhältlich, z. B. unter den Handelsnamen Simulgel^{®} EG, Sepiplus^{®} 400 und Simulgel^{®} EPG von Seppic.

Bevorzugt ist das mindestens eine teilweise oder vollständig neutralisierte, vernetzte Copolymer aus 2-Methyl-2[(1-oxo-2-propenyl)amino]-1-propansulfonsäure (AMPS) und mindestens einem weiteren Monomer, ausgewählt aus Acrylsäure und/oder einem Acrylsäuresalz in einer Gesamtmenge von 0,1 - 1,0 Gew.-%, besonders bevorzugt von 0,2 - 0,6 Gew.-%, außerordentlich bevorzugt von 0,3 - 0,5 Gew.-%, jeweils bezogen auf die gesamte Zusammensetzung, enthalten.

Besonders bevorzugt ist das mindestens eine teilweise oder vollständig neutralisierte, vernetzte Copolymer aus 2-Methyl-2[(1-oxo-2-propenyl)amino]-1-propansulfonsäure (AMPS) und Natriumacrylat in einer Gesamtmenge von 0,1 - 1,0 Gew.-%, besonders bevorzugt von 0,2 - 0,6 Gew.-%, außerordentlich bevorzugt von 0,3 - 0,5 Gew.-%, jeweils bezogen auf die gesamte Zusammensetzung, enthalten.

Überraschend wurde gefunden, dass die erfindungsgemäße Kombination aus dem Protein-basierten Emulgatorsystem, 13 - 20 Gew.-% Konsistenzgeber und Polymerverdickersystem aus mindestens einem teilweise oder vollständig neutralisierten, vernetzten Copolymer aus 2-Methyl-2[(1-oxo-2-propenyl)amino]-1-propansulfonsäure (AMPS) und Vinylpyrrolidon, und mindestens einem teilweise oder vollständig neutralisierten, vernetzten Copolymer aus 2-Methyl-2[(1-oxo-2-propenyl)amino]-1-propansulfonsäure (AMPS) und mindestens einem weiteren Monomer, ausgewählt aus Acrylsäure und/oder einem Acrylsäuresalz, zu Öl-in-Wasser-Emulsionen führt, die die gestellten Aufgaben besonders gut lösen und die insbesondere leicht auf die Haut aufzutragen, aber auch wieder einfach zu entfernen ist, vor allem in größerer Schichtdicke gut auf der Haut haftet und weder heruntertropft noch in die Augen migriert, gleichzeitig eine hohe Lager- und Temperaturstabilität aufweist und - dank des Protein-basierten Emulgators - hervorragend hautverträglich ist und außerdem ein hervorragendes Hautgefühl sowohl bei und als auch nach der Anwendung hervorruft. Die erfindungsgemäßen Öl-in-Wasser-Emulsionen sind daher hervorragend als Pflegemaske, also für einen längeren Verbleib auf der Haut, geeignet. Darüber hinaus sind die erfindungsgemäßen Öl-in-Wasser-Emulsionen auch hervorragend als Vehikel für kosmetische Wirkstoffe, insbesondere für Antifalten-Wirkstoffe, geeignet.
Bevorzugte Polysaccharide sind ausgewählt aus Celluloseethern, vor allem Hydroxyalkylcellulosen, insbesondere Hydroxypropylcellulose, Hydroxypropylmethylcellulose, Hydroxyethylcellulose, Carboxymethylcellulose, Cetylhydroxyethylcellulose, Cetylcellulose, Hydroxybutylmethylcellulose, Methylhydroxyethylcellulose, weiterhin ausgewählt aus Xanthan-Gum, Dehydroxanthan-Gum, Sclerotium Gum, Succinoglucanen, Polygalactomannanen, insbesondere Guar-Gums und Johannisbrotkernmehl (Locust Bean Gum), insbesondere Guar-Gum und Locust Bean Gum selbst und den nichtionischen Hydroxyalkylguarderivaten und Johannisbrotkernmehl-Derivaten, wie Hydroxypropylguar, Carboxymethylhydroxypropylguar, Hydroxypropylmethylguar, Hydroxyethylguar und Carboxymethylguar, weiterhin ausgewählt aus Pectinen, Leinsamen-Gums, Agar-Agar, Carragheen (Carrageenan), Traganth, Gummi arabicum, Karayagummi, Taragummi, Gellan, Propylenglycolalginat, Alginsäuren und deren Salzen (Alginaten), insbesondere Natriumalginaten, Kaliumalginaten und Calciumalginaten, weiterhin - und insbesondere bevorzugt - ausgewählt aus physikalisch (z. B. durch Vorverkleisterung) und/oder chemisch modifizierten Stärken, insbesondere hydroxypropylierten Stärkephosphaten und Octenylstärkesuccinaten und Dodecylstärkesuccinaten und deren Aluminium-, Calcium- oder Natriumsalzen.

Besonders bevorzugt sind hydrophob modifizierte Polysaccharide, insbesondere Cetylhydroxyethylcellulose, Cetylcellulose und die Octenylstärkesuccinate und Dodecylstärkesuccinate und insbesondere die Aluminium-, Calcium- oder Natriumsalze der Succinatstärkederivate. Außerordentlich bevorzugt sind Natriumoctenylstärkesuccinat und Aluminiumoctenylstärkesuccinat.

Das mindestens eine Polysaccharid, insbesondere das mindestens eine hydrophob modifizierte Polysaccharid, insbesondere Aluminiumoctenylstärkesuccinat, ist/sind in einer Gesamtmenge von 0,1 - 2 Gew.-%, bevorzugt 0,5 - 1,5 Gew.-%, besonders bevorzugt 1-1.3 Gew.-%, jeweils bezogen auf die gesamte Zusammensetzung, enthalten.

Durch das erfindungsgemäße Polymerverdickersystem aus den ausgewählten AMPS-Copolymer-Kombinationen konnten die Anwendungseigenschaften für den Fachmann in überraschender Weise entscheidend verbessert werden. Eine weitere überraschende Verbesserung der Konsistenz und des Applikationsverhaltens, insbesondere eine Reduktion der Migration der Zusammensetzung, wurde durch den Zusatz des Polysaccharids, insbesondere des hydrophob modifizierten Polysaccharids, erzielt. Die unerwünschte Migration bezieht sich insbesondere auf das Kriechen der Zusammensetzung in die Augen.

Weitere erfindungsgemäß bevorzugte Zusammensetzungen sind dadurch gekennzeichnet, dass kein Carbomer und kein Acrylates/C 10-30 Alkyl Acrylate Crosspolymer enthalten ist.

Überraschend wurde festgestellt, dass diese Verdicker den Anwendungseigenschaften der erfindüngsgemäß verwendeten Zusammensetzungen eher abträglich wären, daher sollte bevorzugt auf diese Verdicker verzichtet werden.

Weitere erfindungsgemäß bevorzugt verwendete Zusammensetzungen sind dadurch gekennzeichnet, dass mindestens 6 Gew.-%, bezogen auf die gesamte Zusammensetzung, mindestens eines wasserlöslichen Polyols, ausgewählt aus wasserlöslichen Diolen (zweiwertigen Alkoholen), Triolen (zweiwertigen Alkoholen) und höherwertigen Alkoholen sowie Polyethylenglycolen, sowie Mischungen hiervon, enthalten sind.

Bevorzugt sind diese Komponenten ausgewählt aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,2-Hexandiol und 1,6-Hexandiol, Hexantriolen wie 1,2,6-Hexantriol, 1,2-Octandiol, 1,8-Octandiol, 1,2-Decandiol, 1,10-Decandiol, Dipropylenglycol, Tripropylenglycol, technische Oligoglyceringemische mit einem Eigenkondensationsgrad von 1,5 bis 10 wie etwa technische Diglyceringemische mit einem Diglyceringehalt von 40 bis 50 Gew.-% oder Triglycerin, Diglycerin, Triglycerin, Erythrit, Sorbit, Polyethylenglycole (PEG) mit einem durchschnittlichen Molekulargewicht von 100 bis 1000 Dalton, beispielsweise PEG-400, PEG-600 oder PEG-1000 sowie Mischungen der vorgenannten Substanzen. Bevorzugte wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind. Auch Zucker, insbesondere C₄-, C₅- und C₆-Monosaccharide, wie Fructose, Glucose, Maltose, Sucrose, Trehalose und Xylose, und bestimmte Zuckerderivate, insbesondere die entsprechenden Zuckeralkohole Maltitol, Mannit, Sorbit und Inosit, sind erfindungsgemäß bevorzugt.

Bevorzugt ist das mindestens eine wasserlösliche Polyol in einer Gesamtmenge von 7 - 30 Gew.-%, besonders bevorzugt 15 - 25 Gew.-%, enthalten, jeweils bezogen auf die gesamte Zusammensetzung.

Weitere erfindungsgemäß bevorzugt verwendete Zusammensetzungen sind dadurch gekennzeichnet, dass Öl-in-Wasser-Emulgatoren mit einem HLB-Wert von mindestens 10 in einer Gesamtmenge von maximal 0,5 Gew.-%, bevorzugt maximal 0,2 Gew.-%, besonders bevorzugt 0 Gew.-%, jeweils bezogen auf die gesamte Zusammensetzung, enthalten sind.
In der nachfolgenden Tabelle sind verschiedene Öl-in-Wasser-Emulgatoren und Wasser-in-Öl-Emulgatoren und ihre HLB-Werte zusammengestellt. Die HLB-Werte können aber auch nach Griffin berechnet werden, wie es beispielsweise im RÖMPP Chemie Lexikon, insbesondere in der Online-Version von November 2003, und den dort unter dem Stichwort "HLB-System" zitierten Handbüchern von Fiedler, Kirk-Othmer und Janistyn dargestellt beziehungsweise tabelliert ist. Sofern es in der Literatur unterschiedliche Angaben zum HLB-Wert einer Substanz gibt, sollte derjenige HLB-Wert für die erfindungsgemäße Lehre genutzt werden, der dem nach Griffin berechneten Wert am nächsten kommt. Falls sich auf diese Art und Weise kein eindeutiger HLB-Wert ermitteln lässt, ist der HLB-Wert, den der Hersteller des Emulgators angibt, für die erfindungsgemäße Lehre zu nutzen. Falls auch dies nicht möglich ist, ist der HLB-Wert experimentell zu ermitteln.

### HLB-WertChemische Bezeichnung

| | |
|---|---|
| 1 | Triglyceride gesättigter Fettsäuren |
| | Glyceryltrioleat |
| 1,5 | Ethylenglycoldistearat |
| 1,6 | Pur-Cellinöl |
| 1,8 | Sorbitantrioleat |
| | Glycerindioleat |
| 2,1 | Sorbitantristearat |
| 2,4 | Propylenglycollactostearat |
| 2,7 | Glycerinmonooleat |
| | Sorbitdioleat |
| 2,8 | Glycerinmonostearat |
| | Propylenglycolmono-/distearat, nicht selbstemulgierend |
| 2,9 | Ethylenglycolmonostearat |
| 3,0 | Decaglycerindecaoleat |
| | Decaglycerindecastearat |
| | Generol 122 (Rapeseed Sterols) |
| | Sucrosedistearat |
| 3,1 | Decaglycerindecaoleat |
| | Glycerylmonoricinoleat |
| | Pentaerythritylmonostearat |
| | Pentaerythritylsesquioleat |
| 3,2 | Ethylenglycolmonodistearat, nicht selbstemulgierend |
| | Glycolstearat |
| 3,3 | Glycerinmonolaurat |
| 3,4 | Propylenglycolmonostearat |
| 3,5 | Ethylenglycolmonostearat |
| | Pentaerythritylmonooleat |
| | Polyethylenglycol(100)monooleat |
| 3,6 | Glycerinmono-/dioleat, nicht selbstemulgierend |
| | Monoethoxylaurylether |
| 3,7 | Sorbitansesquioleat (Dehymuls SSO) |
| 3,8 | Glycerinmonodistearat, nicht selbstemulgierend |
| | Polyethylenglycol(100)monostearat |
| | Diglycerinsesquioleat |
| | N, N-Dimethylcaproamid |
| | Pentaerythritmonotallat |
| | Propylenglycolmonolaurat |
| 4,0 | Decaglycerinoctaoleat |
| 4,3 | Sorbitanmonooleat (Dehymuls SMO) |
| | Diethylenglycolmonostearat |
| 4,4 | 1.2-Propylenglycolmonodistearat, selbstemulgierend |
| 4,5 | Glycerinmonostearatpalmitat (90 %), nicht selbstemulgierend |
| | Propylenglycolmonolaurat |
| 4,7 | Sorbitanmonostearat (Dehymuls SMS) |
| | Diethylenglycolmonooleat |
| 4,8 | Pentaerythritmonolaurat |
| 4,9 | Polyoxyethylen(2)oleylalkohol (Polyoxyethylen(2)oleylether) |
| | Polyoxyethylen(2)stearylalkohol (Polyoxyethylen(2)stearylether) |
| 5,0 | Ethylenglycolmonodistearat |
| | Generol 122 E 5 (PEG-5 Soy Sterol) |
| | Polyethylenglycol(100)monoricinoleat |
| | Polyethylenglycol(200)distearat |
| | Polyglyceryl-3-isostearate (z. B. Isolan Gl 34 von Tego) |
| 5,9 | Polyethylenglycol(200)dilaurat |
| 6,0 | Decaglycerintetraoleat |
| | Polyethylenglycol(100)monolaurat |
| | Polyethylenglycol(200)dioleat |
| 6,1 | Diethylenglycolmonolaurat (Diglycollaurat) |
| 6,3 | Polyethylenglycol(300)dilaurat |
| 6,4 | Glycerinmonoricinoleat |
| | Glycerinsorbitanmonolaurat |
| 6,5 | Diethylenglycolmonolaurat |
| | Natriumstearoyl-2-lactylat |
| 6,7 | Sorbitanmonopalmitat |
| 6,8 | Glycerinmonococoat |
| | Glycerinmonolaurat |
| 7,0 | Polyoxyethylen(2)C₁₀-C₁₄-fettalkoholether, Laureth-2 (Dehydol LS 2) |
| | Saccharosedistearat |
| 7,2 | Polyethylenglycol(400)dioleat |
| | Saccharosedioleat |
| 7,4 | Polyethylenglycol(100)monolaurat |
| | Saccharosedipalmitat |
| 7,5 | Saccharosedipalmitat |
| 7,6 | Glycerinsorbitanlaurat |
| 7,8 | Polyethylenglycol(400)distearat |
| 7,9 | Polyethylenglycol(200)monostearat |
| | Polyoxyethylen(3)tridecylalkohol |
| 8-8,2 | Polyethylenglycol(400)distearat |
| 8,0 | Polyoxyethylen(3)C₁₀-C₁₄-fettalkoholether, Laureth-3 (Dehydol LS 3) |
| | N.N-Dimethyllauramid |
| | Natriumlauroyllactylat, Natriumlauroyl-2-lactylat |
| | Polyethylenglycol(200)monooleat |
| | Polyethylenglycol(220)monotallat |
| | Polyethylenglycol(1500)dioleat |
| | Polyoxyethylen(4)oleylalkohol |
| | Polyoxyethylen(4)stearylcetylether |
| 8,2 | Triglycerinmonooleat |
| 8,3 | Diethylenglycolmonolaurat |
| 8,4 | Polyoxyethylen(4)cetylether |
| | Polyoxyethylenglycol(400)dioleat |
| 8,5 | Natriumcaproyllactylat |
| | Polyethylenglycol(200)monostearat |
| | Sorbitanmonooleat |
| 8,6 | Sorbitanmonolaurat (Dehymuls SML) |
| | Polyethylenglycol(200)monolaurat |
| 8,8 | Polyoxyethylen(4)myristylether |
| | Polyethylenglycol (400)dioleat |
| 8,9 | Nonylphenol, polyoxyethyliert mit 4 Mol EO |
| 9,0 | Oleth-5 (z. B. Eumulgin O 5) |
| 9,2 - 9,7 | Polyoxyethylen(4)laurylalkohol (je nach Handelsprodukt, z. B. Brij 30, |
| | Dehydol LS 4) |
| 9,3 | Polyoxyethylen(4)tridecylalkohol |
| 9,6 | Polyoxyethylen(4)sorbitanmonostearat |
| 9,8 | Polyethylenglycol (200)monolaurat |
| 10-11 | Polyethylenglycol(400)monooleat |
| 10,0 | Didodecyldimethylammoniumchlorid |
| 10,0 | Polyethylenglycol(200)monolaurat |
| | Polyethylenglycol(400)dilaurat |
| | Polyethylenglycol(600)dioleat |
| | Polyoxyethylen(4)sorbitanmonostearat |
| | Polyoxyethylen(5)sorbitanmonooleat |
| 10,2 | Polyoxyethylen(40)sorbitol hexaoleat |
| 10,4 -10,6 | Polyoxyethylenglycol(600)distearat |
| 10,5 | Polyoxyethylen(20)sorbitantristearat |
| 10,6 | Saccharosemonostearat |
| 10,7 | Saccharosemonooleat |
| 11 - 11,4 | Polyethylenglycol(400)monooleat |
| 11,0 | Polyethylenglycol(350)monostearat |
| | Polyethylenglycol(400)monotallat |
| | Polyoxyethylenglycol(7)monostearat |
| | Polyoxyethylenglycol(8)monooleat |
| | Polyoxyethylen(20)sorbitantrioleat |
| | Polyoxyethylen(6)tridecylalkohol |
| 11,1 | Polyethylenglycol(400)monostearat |
| 11,2 | Polyoxyethylen(9)monostearat |
| | Saccharosemonooleat |
| | Saccharosemonostearat |
| 11,4 | Polyoxyethylen(50)sorbitol hexaoleat |
| | Saccharosemonotallat |
| | Saccharosestearatpalmitat |
| 11,6 | Polyoxyethylenglycol(400)monoricinoleat |
| 11,7 | Saccharosemonomyristat |
| | Saccharosemonopalmitat |
| 12,0 | PEG-10 Soy Sterol (z. B. Generol 122 E 10) |
| | Triethanolaminoleat |
| 12,2-12,3 | Nonylphenol, ethoxyliert mit 8 Mol EO |
| 12,2 | Saccharosemonomyristat |
| 12,4 | Saccharosemonolaurat |
| | Polyoxyethylen(10)oleylalkohol, Polyoxyethylen(10)oleylether |
| | Polyoxyethylen(10)stearylalkohol, Polyoxyethylen(10)stearylether |
| 12,5 | Polyoxyethylen(10)stearylcetylether |
| 12,7 | Polyoxyethylen(8)tridecylalkohol |
| 12,8 | Polyoxyethylenglycol(400)monolaurat |
| | Saccharosemonococoat |
| 12,9 | Polyoxyethylen(10)cetylether |
| 13 | Glycerinmonostearat, ethoxyliert (20 Mol EO) |
| 13,0 | Eumulgin O 10 |
| | Eumulgin 286 |
| | Eumulgin B 1 (Ceteareth-12) |
| 13,0 | C12-Fettamine, ethoxyliert (5 Mol EO) |
| 13,1 | Nonylphenol, ethoxyliert (9,5 Mol EO) |
| 13,2 | Polyethylenglycol(600)monostearat |
| | Polyoxyethylen(1 6)tallöl |
| 13,3 | Polyoxyethylen(4)sorbitanmonolaurat |
| 13,5 | Nonylphenol, ethoxyliert (10,5 Mol EO) |
| | Polyethylenglycol(600)monooleat |
| 13,7 | Polyoxyethylen(10)tridecylalkohol |
| | Polyethylenglycol(660)monotallat |
| | Polyethylenglycol(1500)monostearat |
| | Polyoxyethylenglycol(1500)dioleat |
| 13,9 | Polyethylenglycol(400)monococoat |
| | Polyoxyethylen(9)monolaurat |
| 14-16 | Eumulgin HRE 40 (Ricinusöl, mit 40 EO ethoxyliert und hydriert) |
| 14,0 | Polyoxyethylen(12)laurylether |
| | Polyoxyethylen(1 2)tridecylalkohol |
| 14,2 | Polyoxyethylen(15)stearylalkohol |
| 14,3 | Polyoxyethylen(1 5)stearylcetylether |
| 14,4 | Gemisch aus C12-C15-Fettalkoholen mit 12 Mol EO |
| 14,5 | Polyoxyethylen(12)laurylalkohol |
| 14,8 | Polyoxyethylenglycol(600)monolaurat |
| 14,9 - 15,2 | Sorbitanmonostearat, mit 20 EO ethoxyliert (z. B. Eumulgin SMS 20) |
| 15 - 15,9 | Sorbitanmonooleat, mit 20 EO ethoxyliert (z. B. Eumulgin SMO 20) |
| 15,0 | PEG-20 Glyceryl stearate (z. B. Cutina E 24) |
| | PEG-40 Castor Oil (z. B. Eumulgin RO 40) |
| | Decylglucosid (Oramix NS 10) |
| | Dodecylglucosid (Plantaren APG 600) |
| | Dodecyltrimethylammoniumchlorid |
| | Nonylphenol, ethoxyliert mit 15 Mol EO |
| | Polyethylenglycol(1000)monostearat |
| | Polyoxyethylen(600)monooleat |
| 15-17 | Eumulgin HRE 60 (Ricinusöl, mit 60 EO ethoxyliert und hydriert) |
| 15,3 | C12-Fettamine, polyoxyethyliert mit 12 Mol EO |
| | Polyoxyethylen(20)oleylalkohol, Polyoxyethylen(20)oleylether |
| 15,4 | Polyoxyethylen(20)stearylcetylether (z. B. Eumulgin B 2 (Ceteareth-20)) |
| 15,5 | Polyoxyethylen(20)stearylalkohol |
| 15,6 | Polyoxyethylenglycol(1000)monostearat |
| | Polyoxyethylen(20)sorbitanmonopalmitat |
| 15,7 | Polyoxyethylen(20)cetylether |
| 15,9 | Dinatriumtriethanolamindistearylheptaglycolethersulfosuccinat |
| 16,0 | Nonylphenol ethoxyliert mit 20 Mol EO |
| | Polyoxyethylen(25)propylenglycolstearat |
| 16 - 16,8 | Polyoxyethylen(30)monostearat |
| 16,3-16,9 | Polyoxyethylen(40)monostearat |
| 16,5 - 16,7 | Polyoxyethylen(20)sorbitanmonolaurat (z. B. Eumulgin SML 20) |
| 16,6 | Polyoxyethylen(20)sorbit |
| 16,7 | C18-Fettamine, polyoxyethyliert mit 5 Mol EO |
| | Polyoxyethylen(23)laurylalkohol |
| 17,0 | Ceteareth-30, z. B. Eumulgin B 3 |
| | Octylglucosid (Triton CG 110) |
| | Polyoxyethylen(30)glycerylmonolaurat |
| 17,1 | Nonylphenol, ethoxyliert mit 30 Mol EO |
| 17,4 | Polyoxyethylen(40)stearylalkohol |

Weitere erfindungsgemäß bevorzugt verwendete Zusammensetzungen sind dadurch gekennzeichnet, dass mindestens ein kosmetischer Wirkstoff enthalten ist, der ausgewählt ist aus Monomeren, Oligomeren und Polymeren von Aminosäuren, N-C₂-C₂₄-Acylaminosäuren, den Estern und/oder den physiologisch verträglichen Metallsalzen dieser Substanzen,
DNA- oder RNA-Oligonucleotiden,
natürlichen Betainverbindungen,
Vitaminen, Provitaminen und Vitaminvorstufen der Gruppen A, B, C, E und H und den Estern der vorgenannten Substanzen,
Flavonoiden und Flavonoid-reichen Pflanzenextrakten,
Isofiavonoiden und Isoflavonoid-reichen Pflanzenextrakten,
alkyl- oder hydroxyalkylsubstituierten Harnstoffderivaten,
Ubichinon und Ubichinol sowie deren Derivaten,
Silymarin,
natürlich vorkommenden Xanthin-Derivaten, ausgewählt aus Coffein, Theophyllin, Theobromin und Aminophyllin,
Ectoin,
Kreatin,
Olivenblattextrakten, Ursolsäure, Oleanol und/oder Oleanolsäure,
Mono- und Polyhydroxystilbenen, insbesondere Resveratrol, und deren Estern,
Derivaten von methyliertem Silanol,
Phytinsäure,
Extrakten aus Maiskörnern (Zea Mays (Corn) Kernel Extract),
Extrakten aus Haferkörnern (Avena Sativa (Oat) Kernel Extract),
Saccharomyces/Xylinum/Black Tea Ferment,
Apfelkernextrakten (Pyrus Malus (Apple) Fruit Extract),
Lotuskeim-Extrakten (Nelumbo Nucifera Germ Extract),
Rotweinextrakten,
Traubenkernextrakten (Vitis Vinifera (Grape) Seed Extract), die bevorzugt aus der Chardonnay-Traube stammen,
Extrakten aus Schwarzen Holunderblüten (Sambucus Nigra Flower Extract),
Wirkstoffen, die die beta-Endorphinsynthese in Keratinozyten stimulieren,
hautaufhellenden Wirkstoffen,
Wirkstoffen, die die Prostaglandinsynthese und/oder die Leukotrien-Synthese inhibieren, sowie sebumregulierenden Wirkstoffen.

Ein weiterer Gegenstand der vorliegenden Anmeldung ist die Verwendung einer kosmetischen Zusammensetzung in Form einer Öl-in-Wasser-Emulsion, enthaltend
a) ein Emulgatorsystem, umfassend
   a.i) 0,1 -0,3 Gew.-% eines wasserlöslichen Proteins und
   a.ii) 3 - 20 Gew.-% eines Öls, ausgewählt aus pflanzlichen Ölen sowie synthetischen Triglyceriden, synthetischen Propylenglycoldiestern und Fettsäureestern einwertiger linearer oder verzweigter C₂-C₁₈-Alkanole, die unter Normalbedingungen flüssig sind, sowie Mischungen dieser Komponenten,
b) 13 - 20 Gew.-% Konsistenzgeber(n), die unter Normalbedingungen fest sind, ausgewählt aus linearen, gesättigten C₁₂-C₃₀-Alkanolen, Wachsen und Partialestern von C₂ - C₁₀-Polyolen mit C₈-C₃₀-Fettsäuren, sowie Mischungen dieser Komponenten,
c) ein Polymerverdickersystem, umfassend
   c.i) mindestens ein teilweise oder vollständig neutralisiertes, vernetztes Copolymer aus 2-Methyl-2[(1-oxo-2-propenyl)amino]-1-propansulfonsäure (AMPS) und Vinylpyrrolidon,
   c.ii) mindestens ein teilweise oder vollständig neutralisiertes, vernetztes Copolymer aus 2-Methyl-2[(1-oxo-2-propenyl)amino]-1-propansulfonsäure (AMPS) und mindestens
      einem weiteren Monomer, ausgewählt aus Acrylsäure und/oder einem Acrylsäuresalz, als kosmetische Pflegemaske.

## Patentansprüche

1. Nicht-therapeutisches, kosmetisches Verfahren zur Behandlung der Haut, **dadurch gekennzeichnet, dass** eine kosmetische Zusammensetzung in Form einer Öl-in-Wasser-Emulsion, enthaltend
a) ein Emulgatorsystem, umfassend
a.i) 0,1 - 0,3 Gew.-% eines wasserlöslichen Proteins und
a.ii) 3 - 20 Gew.-% eines Öls, ausgewählt aus pflanzlichen Ölen sowie synthetischen Triglyceriden, synthetischen Propylenglycoldiestern und Fettsäureestern einwertiger linearer oder verzweigter C₂-C₁₈-Alkanole, die unter Normalbedingungen flüssig sind, sowie Mischungen dieser Komponenten,
b) 13-20 Gew.-% Konsistenzgeber(n), die unter Normalbedingungen fest sind, ausgewählt aus linearen, gesättigten C₁₂-C₃₀-Alkanolen, Wachsen und Partialestern von C₂ - C₁₀-Polyolen mit C₈-C₃₀-Fettsäuren, sowie Mischungen dieser Komponenten,
c) ein Polymerverdickersystem, umfassend
c.i) mindestens ein teilweise oder vollständig neutralisiertes, vernetztes Copolymer aus 2-Methyl-2[(1-oxo-2-propenyl)amino]-1-propansulfonsäure (AMPS) und Vinylpyrrolidon,
c.ii) mindestens ein teilweise oder vollständig neutralisiertes, vernetztes Copolymer aus 2-Methyl-2[(1-oxo-2-propenyl)amino]-1-propansulfonsäure (AMPS) und mindestens einem weiteren Monomer, ausgewählt aus Acrylsäure und/oder einem Acrylsäuresalz, auf die Haut, insbesondere die Gesichtshaut, aufgetragen wird, dort für eine Zeit von 5 bis 60 Minuten verbleibt und anschließend mit Wasser und/oder einem Substrat, insbesondere einem Lappen, Tuch, Pad oder Bausch, entfernt wird.

2. Verfahren gemäß Anspruch 1, **dadurch gekennzeichnet, dass** weiterhin mindestens ein Polysaccharid enthalten ist.

3. Verfahren gemäß Anspruch 2, **dadurch gekennzeichnet, dass** das Polysaccharid hydrophob modifiziert ist.

4. Verfahren gemäß einem der vorstehenden Ansprüche, **dadurch gekennzeichnet, dass** kein Carbomer und kein Acrylates/C10-30 Alkyl Acrylate Crosspolymer enthalten ist.

5. Verfahren gemäß einem der vorstehenden Ansprüche, **dadurch gekennzeichnet, dass** das mindestens eine teilweise oder vollständig neutralisierte, vernetzte Copolymer aus 2-Methyl-2[(1-oxo-2-propenyl)amino]-1-propansulfonsäure (AMPS) und Vinylpyrrolidon in einer Gesamtmenge von mindestens 0,3 Gew.-%, bezogen auf die gesamte Zusammensetzung, enthalten ist.

6. Verfahren gemäß einem der vorstehenden Ansprüche, **dadurch gekennzeichnet, dass** das mindestens eine teilweise oder vollständig neutralisierte, vernetzte Copolymer aus 2-Methyl-2[(1-oxo-2-propenyl)amino]-1-propansulfonsäure (AMPS) und mindestens einem weiteren Monomer, ausgewählt aus Acrylsäure und/oder einem Acrylsäuresalz, in einer Gesamtmenge von mindestens 0,2 Gew.-%, bezogen auf die gesamte Zusammensetzung, enthalten ist.

7. Verfahren gemäß einem der vorstehenden Ansprüche, **dadurch gekennzeichnet, dass** mindestens 6 Gew.-%, bezogen auf die gesamte Zusammensetzung, mindestens eines wasserlöslichen Polyols, ausgewählt aus wasserlöslichen Diolen (zweiwertigen Alkoholen), Triolen (zweiwertigen Alkoholen) und höherwertigen Alkoholen sowie Polyethylenglycolen, enthalten ist.

8. Verfahren gemäß einem der vorstehenden Ansprüche, **dadurch gekennzeichnet, dass** der mindestens eine Konsistenzgeber, der unter Normalbedingungen fest ist, ausgewählt aus linearen, gesättigten C₁₂-C₃₀-Alkanolen, Wachsen und Partialestern von C₂ - C₁₀-Polyolen mit C₈-C₂₄-Fettsäuren, sowie Mischungen dieser Komponenten, in einer Gesamtmenge von 13 **-** 18 Gew.-%, bevorzugt 15 - 18 Gew.-%, besonders bevorzugt 15 - 17 Gew.-%, jeweils bezogen auf die gesamte Zusammensetzung, enthalten ist.

9. Verfahren gemäß einem der vorstehenden Ansprüche, **dadurch gekennzeichnet, dass** Öl-in-Wasser-Emulgatoren mit einem HLB-Wert von mindestens 10 in einer Gesamtmenge von maximal 0,5 Gew.-%, bevorzugt maximal 0,2 Gew.-%, besonders bevorzugt 0 Gew.-%, jeweils bezogen auf die gesamte Zusammensetzung, enthalten sind.

10. Verfahren gemäß einem der vorstehenden Ansprüche, **dadurch gekennzeichnet, dass** mindestens ein kosmetischer Wirkstoff enthalten ist, der ausgewählt ist aus
a) Monomeren, Oligomeren und Polymeren von Aminosäuren, N-C₂-C₂₄-Acylaminosäuren, den Estern und/oder den physiologisch verträglichen Metallsalzen dieser Substanzen,
b) DNA- oder RNA-Oligonucleotiden,
c) natürlichen Betainverbindungen,
d) Vitaminen, Provitaminen und Vitaminvorstufen der Gruppen A, B, C, E und H und den Estern der vorgenannten Substanzen,
e) Flavonoiden und Flavonoid-reichen Pflanzenextrakten,
f) Isoflavonoiden und Isoflavonoid-reichen Pflanzenextrakten,
g) alkyl- oder hydroxyalkylsubstituierten Harnstoffderivaten,
h) Ubichinon und Ubichinol sowie deren Derivaten,
i) Silymarin,
j) natürlich vorkommenden Xanthin-Derivaten, ausgewählt aus Coffein, Theophyllin, Theobromin und Aminophyllin,
k) Ectoin,
l) Kreatin,
m) Olivenblattextrakten, Ursolsäure, Oleanol und/oder Oleanolsäure,
n) Mono- und Polyhydroxystilbenen, insbesondere Resveratrol, und deren Estern,
o) Derivaten von methyliertem Silanol,
p) Phytinsäure,
q) Extrakten aus Maiskörnern (Zea Mays (Corn) Kernel Extract),
r) Extrakten aus Haferkörnern (Avena Sativa (Oat) Kernel Extract),
s) Saccharomyces/Xylinum/Black Tea Ferment,
t) Apfelkernextrakten (Pyrus Malus (Apple) Fruit Extract),
u) Lotuskeim-Extrakten (Nelumbo Nucifera Germ Extract),
v) Rotweinextrakten,
w) Traubenkernextrakten (Vitis Vinifera (Grape) Seed Extract), die bevorzugt aus der Chardonnay-Traube stammen,
x) Extrakten aus Schwarzen Holunderblüten (Sambucus Nigra Flower Extract),
y) Wirkstoffen, die die beta-Endorphinsynthese in Keratinozyten stimulieren,
z) hautaufhellenden Wirkstoffen,
aa) Wirkstoffen, die die Prostaglandinsynthese und/oder die Leukotrien-Synthese inhibieren, sowie
bb) sebumregulierenden Wirkstoffen.

11. Verwendung einer kosmetischen Zusammensetzung in Form einer Öl-in-Wasser-Emulsion, enthaltend
a) ein Emulgatorsystem, umfassend
a.iii) 0,1 - 0,3 Gew.-% eines wasserlöslichen Proteins und
a.iv) 3-20 Gew.-% eines Öls, ausgewählt aus pflanzlichen Ölen sowie synthetischen Triglyceriden, synthetischen Propylenglycoldiestern und Fettsäureestern einwertiger linearer oder verzweigter C₂-C₁₈-Alkanole, die unter Normalbedingungen flüssig sind, sowie Mischungen dieser Komponenten,
b) 13-20 Gew.-% Konsistenzgeber(n), die unter Normalbedingungen fest sind, ausgewählt aus linearen, gesättigten C₁₂-C₃₀-Alkanolen, Wachsen und Partialestern von C₂ - C₁₀-Polyolen mit C₈-C₃₀-Fettsäuren, sowie Mischungen dieser Komponenten,
c) ein Polymerverdickersystem, umfassend
c.i) mindestens ein teilweise oder vollständig neutralisiertes, vernetztes Copolymer aus 2-Methyl-2[(1-oxo-2-propenyl)amino]-1-propansulfonsäure (AMPS) und Vinylpyrrolidon,
c.ii) mindestens ein teilweise oder vollständig neutralisiertes, vernetztes Copolymer aus 2-Methyl-2[(1-oxo-2-propenyl)amino]-1-propansulfonsäure (AMPS) und mindestens einem weiteren Monomer, ausgewählt aus Acrylsäure und/oder einem Acrylsäuresalz, als kosmetische Pflegemaske.

12. Verwendung gemäß Anspruch 11, **dadurch gekennzeichnet, dass** die kosmetische Zusammensetzung wie in dem Verfahren gemäß einem der Ansprüche 2 -10 zusammengesetzt ist.

## Claims

1. A non-therapeutic, cosmetic method for treating the skin, **characterized in that** a cosmetic composition in the form of a oil-in-water emulsion, containing
a) an emulsifier system, comprising
a.i) 0.1-0.3% by weight of water-soluble protein, and
a.ii) 3-20% by weight of an oil, selected from vegetable oils as well as synthetic triglycerides, synthetic propylene glycol diesters and fatty acid esters of monovalent linear or branched C₂-C₁₈ alkanols, which are liquid under normal conditions, as well as mixtures of these components,
b) 13-20% by weight of consistency regulator(s), which is(are) solid under normal conditions, selected from linear, saturated C₁₂-C₃₀ alkanols, waxes and partial esters of C₂-C₁₀ polyols with C₈-C₃₀ fatty acids, as well as mixtures of these components,
c) a polymeric thickener system, comprising
c.i) at least one partly or completely neutralized crosslinked copolymer of 2-methyl-2[(1-oxo-2-propenyl)amino]-1-propane-sulfonic acid (AMPS) and vinylpyrrolidone,
c.ii) at least one partly or completely neutralized, crosslinked copolymer of 2-methyl-2[(1-oxo-2-propenyl)amino]-1-propane-sulfonic acid (AMPS) and at least one further monomer selected from acrylic acid and/or an acrylic acid salt,
is applied on the skin, in particular facial skin, remains thereon for a period of 5 to 60 minutes and is subsequently removed with water and/or with a substrate, in particular a cloth, towel, pad, or wad.

2. The method according to claim 1, **characterized in that** at least one polysaccharide is further contained.

3. The method according to claim 2, **characterized in that** the polysaccharide is hydrophobically modified.

4. The method according to any of the preceding claims, **characterized in that** no carbomer and no acrylates/C₁₀₋₃₀ alkyl acrylate crosspolymer are contained.

5. The method according to any of the preceding claims, **characterized in that** said at least one partly or completely neutralized crosslinked copolymer of 2-methyl-2[(1-oxo-2-propenyl)amino]-1-propanesulfonic acid (AMPS) and vinylpyrrolidone is contained in a total amount of at least 0.3% by weight based on the total composition.

6. The method according to any of the preceding claims, **characterized in that** said at least one partly or completely neutralized, crosslinked copolymer of 2-methyl-2[(1-oxo-2-propenyl)amino]-1-propanesulfonic acid (AMPS) and at least one further monomer, selected from acrylic acid and/or an acrylic acid salt, is contained in a total amount of at least 0.2% by weight, based on the total composition.

7. The method according to any of the preceding claims, **characterized in that** at least 6% by weight, based on the total composition, of at least one water-soluble polyol, selected from water-soluble diols (bivalent alcohols), triols (trivalent alcohols) and alcohols of higher valency as well as polyethylene glycols, is contained.

8. The method according to any of the preceding claims, **characterized in that** said at least one consistency regulator, which is solid under normal conditions, selected from linear, saturated, C₁₂-C₃₀ alkanols, waxes and partial esters of C₂-C₁₀ polyols with C₈-C₂₄ fatty acids, as well as mixtures of these components, is contained in a total amount from 13 to 18% by weight, preferably 15-18% by weight, more preferably 15-17% by weight, each based on the total composition.

9. The method according to any of the preceding claims, **characterized in that** oil-in-water emulsifiers with an HLB value of at least 10 are contained in a total amount of at most 0.5% by weight, preferably at most 0.2% by weight, more preferably 0% by weight, each based on the total composition.

10. The method according to any of the preceding claims, **characterized in that** at least one cosmetic active ingredient is contained, which is selected from
a) monomers, oligomers and polymers of amino acids, N-(C₂-C₄)-acylamino acids, esters and/or physiologically acceptable metal salts of these substances,
b) DNA or RNA oligonucleotides,
c) natural betaine compounds,
d) vitamins, pro-vitamins and vitamin precursors of the groups A, B, C, E and H and esters of the aforementioned substances,
e) flavonoids and flavonoid-rich plant extracts,
f) isoflavonoids and isoflavonoid-rich plant extracts,
g) alkyl- or hydroxyalkyl-substituted urea derivatives,
h) ubiquinone and ubiquinol as well as their derivatives,
i) silymarin,
j) naturally occurring xanthine derivatives, selected from caffeine, theophylline, theobromine and aminophylline,
k) ectoine,
l) creatine,
m) olive leaf extracts, ursolic acid, oleanol and/or oleanolic acid,
n) mono- and poly-hydroxystilbenes, in particular resveratrol, and their esters,
o) derivatives of methylated silanol,
p) phytic acid,
q) extracts of maize grains (*Zea Mays* (corn) kernel extract),
r) extracts of oat grains (*Avena Sativa* (oat) kernel extract),
s) *Saccharomyces*/*Xylinum*/black tea ferment,
t) apple pip extracts (*Pyrus Malus* (Apple) fruit extract),
u) Lotus germ extracts (*Nelumbo Nucifera* germ extract),
v) red wine extracts,
w) grape seed extracts (*Vitis Vinifera* (grape) seed extract), which preferably stem from Chardonnay vine,
x) extracts of black elderflowers (*Sambucus Nigra* flower extract),
y) active substances which stimulate beta-endorphin synthesis in keratinocytes,
z) skin-lightening active substances,
aa) active substances, which inhibit prostaglandin synthesis and/or leukotriene synthesis, as well as
bb) sebum-regulating active substances.

11. The use of a cosmetic composition in the form of a oil-in-water emulsion, containing
a) an emulsifier system, comprising
a.iii) 0.1-0.3% by weight of water-soluble protein, and
a.iv) 3-20% by weight of an oil, selected from vegetable oils as well as synthetic triglycerides, synthetic propylene glycol diesters and fatty acid esters of monovalent linear or branched C₂-C₁₈ alkanols, which are liquid under normal conditions, as well as mixtures of these components,
b) 13-20% by weight of consistency regulator(s), which is(are) solid under normal conditions, selected from linear, saturated C₁₂-C₃₀ alkanols, waxes and partial esters of C₂-C₁₀ polyols with C₈-C₃₀ fatty acids, as well as mixtures of these components,
c) a polymeric thickener system, comprising containing
c.i) at least one partly or completely neutralized, crosslinked copolymer of 2-methyl-2[(1-oxo-2-propenyl)amino]-1-propane-sulfonic acid (AMPS) and vinylpyrrolidone,
c.ii) at least one partly or completely neutralized, crosslinked copolymer of 2-methyl-2[(1-oxo-2-propenyl)amino]-1-propane-sulfonic acid (AMPS) and at least one further monomer, selected from acrylic acid and/or an acrylic acid salt, as a cosmetic care mask.

12. The use according to claim 11, **characterized in that** the cosmetic composition is composed as in the method according to any of claims 2 to 10.

## Revendications

1. Procédé cosmétique, non thérapeutique, pour le traitement de la peau, **caractérisé en ce qu'**une composition cosmétique sous forme d'une émulsion huile-dans-eau, contenant
a) un système d'émulsifiant, comprenant
a.i) 0,1-0,3% en poids d'une protéine soluble dans l'eau et
a.ii) 3-20% en poids d'une huile, choisie parmi les huiles végétales ainsi que les triglycérides synthétiques, les diesters de propylèneglycol synthétiques et les esters d'acides gras d'alcanols en C₂-C₁₈ monovalents, linéaires ou ramifiés, qui sont liquides dans les conditions normales, ainsi que les mélanges de ces composants,
b) 13-20% en poids d'agent(s) conférant une consistance, qui est/sont solide(s) dans les conditions normales, choisi(s) parmi les alcanols en C₁₂-C₃₀ linéaires, saturés, les cires et les esters partiels de polyols en C₂-C₁₀ avec des acides gras en C₈-C₃₀ ainsi que les mélanges de ces composants,
c) un système épaississant polymère, comprenant
c.i) au moins un copolymère réticulé, partiellement ou totalement neutralisé d'acide 2-méthyl-2[(1-oxo-2-propényl)amino]-1-propanesulfonique (AMPS) et de vinylpyrrolidone,
c.ii) au moins un copolymère réticulé, partiellement ou totalement neutralisé d'acide 2-méthyl-2[(1-oxo-2-propényl)amino]-1-propanesulfonique (AMPS) et d'au moins un autre monomère, choisi parmi l'acide acrylique et/ou un sel d'acide acrylique,
est appliquée sur la peau, en particulier sur la peau du visage, y reste pendant un laps de temps de 5 à 60 minutes, puis est éliminée avec de l'eau et/ou un substrat, en particulier un chiffon, un tissu, un coton ou un tampon.

2. Procédé selon la revendication 1, **caractérisé en ce qu'**au moins un polysaccharide est en outre contenu.

3. Procédé selon la revendication 2, **caractérisé en ce que** le polysaccharide est modifié de manière hydrophobe.

4. Procédé selon l'une quelconque des revendications précédentes, **caractérisé en ce que** ni un carbomère, ni un copolymère réticulé d'acrylates/acrylate d'alkyle en C₁₀₋₃₀ n'est contenu.

5. Procédé selon l'une quelconque des revendications précédentes, **caractérisé en ce que** ledit au moins un copolymère réticulé, partiellement ou totalement réticulé d'acide 2-méthyl-2[(1-oxo-2-propényl)amino]-1-propanesulfonique (AMPS) et de vinylpyrrolidone est contenu en une quantité totale d'au moins 0,3% en poids, par rapport à la composition totale.

6. Procédé selon l'une quelconque des revendications précédentes, **caractérisé en ce que** ledit au moins un copolymère réticulé, partiellement ou totalement neutralisé d'acide 2-méthyl-2[(1-oxo-2-propényl)amino)-1-propanesulfonique (AMPS) et d'au moins un autre monomère, choisi parmi l'acide acrylique et/ou un sel d'acide acrylique, est contenu en une quantité totale d'au moins 0,2% en poids par rapport à la composition totale.

7. Procédé selon l'une quelconque des revendications précédentes, **caractérisé en ce qu'**au moins 6% en poids, par rapport à la composition totale, d'au moins un polyol soluble dans l'eau, choisi parmi les diols (alcools divalents) solubles dans l'eau, les triols (alcools trivalents) solubles dans l'eau et les alcools de valence supérieure solubles dans l'eau ainsi que les polyéthylèneglycols, est contenu.

8. Procédé selon l'une quelconque des revendications précédentes, **caractérisé en ce que** ledit au moins un agent conférant une consistance, qui est solide dans des conditions normales, est choisi parmi les alcanols en C₁₂-C₃₀, saturés, linéaires, les cires et les esters partiels de polyols en C₂-C₁₀ avec des acides gras en C₈-C₂₄ ainsi que les mélanges de ces composés, est contenu en une quantité totale de 13-18% en poids, de préférence de 15-18% en poids, de manière particulièrement préférée de 15-17% en poids, à chaque fois par rapport à la composition totale.

9. Procédé selon l'une quelconque des revendications précédentes, **caractérisé en ce que** des émulsifiants huile-dans-eau présentant une valeur BHL d'au moins 10 sont contenus en une quantité totale d'au maximum 0,5% en poids, de préférence d'au maximum 0,2% en poids, de manière particulièrement préférée de 0% en poids, à chaque fois par rapport à la composition totale.

10. Procédé selon l'une quelconque des revendications précédentes, **caractérisé en ce qu'**au moins une substance active cosmétique est contenue, qui est choisie parmi
a) les monomères, oligomères et polymères d'aminoacides, de N-C₂-C₂₄-acylaminoacides, les esters et/ou les sels métalliques physiologiquement compatibles de ces substances,
b) les oligonucléotides d'ADN ou d'ARN,
c) les composés naturels de type bétaïne,
d) les vitamines, les provitamines et les précurseurs de vitamines, des groupes A, B, C, E, et H et les esters des substances susmentionnées,
e) les flavonoïdes et les extraits végétaux riches en flavonoïdes,
f) les isoflavonoïdes et les extraits végétaux riches en isoflavonoïdes,
g) les dérivés d'urée substitués par alkyle ou hydroxyalkyle,
h) l'ubiquinone et l'ubiquinol ainsi que leurs dérivés,
i) le silymarin,
j) les dérivés naturels de xanthine, choisis parmi la caféine, la théophylline, la théobromine et l'aminophylline,
k) l'ectoïne,
l) la créatine,
m) les extraits de feuilles d'olivier, l'acide ursolique, l'oléanol et/ou l'acide oléanolique,
n) les monohydroxystilbènes et les polyhydroxystilbènes, en particulier le resvératrol, et ses esters,
o) les dérivés du silanol méthylé,
p) l'acide phytique,
q) les extraits de grains de maïs (Zea Mays (Corn) Kernel Extract),
r) les extraits de grains d'avoine (Avena Sativa (Oat) Kernel Extract),
s) le ferment de saccharomyces/xylinum/thé noir (Saccharomyces/Xylinum/Black Tea Ferment),
t) les extraits de pépins de pomme (Pyrus Malus (Apple) Fruit Extract),
u) les extraits de germes de lotus (Nelumbo Nucifera Germ Extract),
v) les extraits de vin rouge
w) les extraits de pépins de raisin (Vitis Vinifera (Grape) Seed Extract), qui proviennent de préférence du raisin Chardonnay,
x) les extraits de fleurs de sureau noir (Sambucus Nigra Flower Extract),
y) les substances actives qui stimulent la synthèse de bêta-endorphines dans les kératinocytes,
z) les substances actives d'éclaircissement de la peau,
aa) les substances actives qui inhibent la synthèse de la prostaglandine et/ou des leucotriènes, ainsi que
bb) les substances actives régulant le sébum.

11. Utilisation d'une composition cosmétique sous forme d'une émulsion huile-dans-eau, contenant
a) un système d'émulsifiant, comprenant
a.iii) 0,1-0,3% en poids d'une protéine soluble dans l'eau et
a.iv) 3-20% en poids d'une huile, choisie parmi les huiles végétales ainsi que les triglycérides synthétiques, les diesters de propylèneglycol synthétiques et les esters d'acides gras d'alcanols en C₂-C₁₈ monovalents, linéaires ou ramifiés, qui sont liquides dans les conditions normales, ainsi que les mélanges de ces composants,
b) 13-20% en poids d'agent(s) conférant une consistance, qui est/sont solide(s) dans les conditions normales, choisi(s) parmi les alcanols en C₁₂-C₃₀ linéaires, saturés, les cires et les esters partiels de polyols en C₂-C₁₀ avec des acides gras en C₈-C₃₀ ainsi que les mélanges de ces composants,
c) un système épaississant polymère, comprenant
c.i) au moins un copolymère réticulé, partiellement ou totalement neutralisé d'acide 2-méthyl-2[(1-oxo-2-propényl)amino]-1-propanesulfonique (AMPS) et de vinylpyrrolidone,
c.ii) au moins un copolymère réticulé, partiellement ou totalement neutralisé d'acide 2-méthyl-2[(1-oxo-2-propényl)amino]-1-propanesulfonique (AMPS) et d'au moins un autre monomère, choisi parmi l'acide acrylique et/ou un sel d'acide acrylique,
comme masque de soin cosmétique.

12. Utilisation selon la revendication 11, **caractérisée en ce que** la composition cosmétique est composée comme dans le procédé selon l'une quelconque des revendications 2-10.
